# EUROPEAN PATENT APPLICATION

(11) **EP 4 583 113 A1**
(43) Date of publication of application: **09.07.2025**
(21) Application number: 23880193.0
(22) Date of filing: 17.10.2023
(51) Int. Cl.: G16C 20/30, G16C 20/20, G16C 20/70

(54) **ARTIFICIAL INTELLIGENCE-BASED DEVICE, METHOD, AND PROGRAM FOR PREDICTING PHYSICAL PROPERTY OF MIXTURE**

(30) Priority: 18.10.2022 KR 20220134293
(71) Applicant: LG Management Development Institute Co., Ltd., Seoul 07336 (KR); LG Energy Solution, Ltd., Seoul 07335 (KR)
(72) Inventor: PARK, Changyoung, Seoul 07796 (KR); YANG, Hongjun, Seoul 07796 (KR); LEE, Jaewan, Seoul 07796 (KR); HAN, Sehui, Seoul 07796 (KR); JEON, Hyelim, Daejeon 34122 (KR); LEE, Boram, Daejeon 34122 (KR); LEE, Changhoon, Daejeon 34122 (KR); KIM, Hyung Tae, Daejeon 34122 (KR)
(74) Representative: BCKIP Part mbB
(86) International application number: PCT/KR2023/016080
(87) International publication number: WO 2024/085616

(57) **Abstract**

A device for predicting the physical properties of a mixture including a plurality of materials is disclosed. The device may comprise a memory in which a first AI model trained to output first feature data of material information, and a second AI model trained to output physical property prediction information of the first feature data are stored, and a processor for executing the first AI model and the second AI model, wherein the processor may input, into the first AI model, material information of each of the plurality of materials to acquire first feature data of each of the plurality of materials, and input the first feature data into the second AI model to acquire physical property prediction information of the mixture.

## Description

### [Technical Field]

The present disclosure relates to a device, method, and program for analyzing a mixture. More specifically, the present disclosure relates to an artificial intelligence-based device, method, and program for predicting physical properties of the mixture.

### [Background Art]

Recently, as consumer demands and demand trends have diversified and product development methods have diversified, the development of a new mixture that can be used in product manufacturing is actively underway. In order to more efficiently develop and mass-produce the new mixture that can be used in product manufacturing, prediction and analysis of the physical properties of the corresponding mixture are essential.

Conventionally, technologies for predicting or analyzing physical properties for a single material or an alloy were developed and utilized. However, as the number of materials constituting the corresponding mixture increases and a mixing ratio of each material becomes more complex, the conventional technologies face a limitation that it is difficult to efficiently predict the physical properties of the corresponding mixture. Accordingly, there is an increasing need for a more efficient method for predicting the physical properties of a mixture.

### [Disclosure]

### [Technical Problem]

Embodiments disclosed in the present disclosure are directed to providing an artificial intelligence-based device, method, and program for predicting physical properties of a mixture.

Objects of the present disclosure are not limited to the above-described object, and other objects that are not mentioned will be clearly understood by those skilled in the art from the following description.

### [Technical Solution]

According to an aspect of the present disclosure, there is provided a device for predicting physical properties of a mixture including a plurality of materials, which may include a memory in which a first AI model trained to output first feature data associated with material information, and a second AI model trained to output physical property prediction information associated with the first feature data are stored, and a processor configured to execute the first AI model and the second AI model, wherein the processor may be configured to obtain first feature data associated with each of the plurality of materials by inputting, into the first AI model, material information of each of the plurality of materials, and obtain physical property prediction information associated with the mixture by inputting the first feature data into the second AI model.

In addition, the first AI model may include an attention-based model, and the attention-based model may be trained to extract feature data of a specific material based on at least one of polarizability information and hydrophobicity information of the specific material.

In addition, the first AI model may include a molecular contrastive learning-based model, and the molecular contrastive learning-based model may be trained to align molecules with similar structures on a latent space.

In addition, the first AI model may be trained to update second feature data extracted from two-dimensional graph information of a specific material based on third feature data extracted from three-dimensional structural characteristics of the specific material, and may be trained to output the first feature data based on the updated data.

In addition, the three-dimensional structural characteristics of the specific material may include a plurality of spatial arrangement conformers of the specific material.

In addition, the second AI model may include a multi-layer perceptron-based model.

In addition, the second AI model may include a transformer encoder model.

In addition, the processor may obtain physical property prediction information associated with the mixture by inputting the first feature data and component ratio information of the plurality of materials in the mixture into the second AI model.

In addition, the first AI model and the second AI model may be trained in an end-to-end training manner.

In addition, the material information may include chemical information, and the chemical information may include molecular information (e.g., a simplified molecular-input line-entry system (SMILES), an international chemical identifier (INCHI), or a self-referencing embedded string (SELFIES)) of each of the plurality of materials.

In addition, the chemical information may include at least one of atom properties or bond properties related to the mixture. As another example, at least one of the atomic property or bond property related to the mixture may be obtained based on the molecular information.

In addition, the first feature data may include molecular feature data of each of the plurality of materials.

In addition, the physical property prediction information may include Gibbs free energy prediction information.

In addition, as another embodiment of the present disclosure, a method for predicting physical properties of a mixture including a plurality of materials, performed by a device, the method may include obtaining first feature data associated with each of the plurality of materials by inputting material information of each of the plurality of materials into a first AI model that has trained first feature data associated with material information, and obtaining physical property prediction information associated with the mixture by inputting the first feature data into a second AI model that has trained physical property prediction information associated with the first feature data.

Additionally, a computer program that is stored in a computer-readable recording medium for implementing the present disclosure may further be provided.

Additionally, a computer-readable recording medium that records a computer program to execute a method for implementing the present disclosure may further be provided.

### [Advantageous Effects]

According to the above configuration of the present disclosure, an artificial intelligence-based device, method, and program for predicting physical properties of a mixture can be provided.

In addition, according to the above configuration of the present disclosure, the physical properties of the mixture can be more efficiently predicted by using an artificial intelligence model that can consider interactions between components of the mixture.

Effects of the present disclosure are not limited to the above effects, and other effects that are not mentioned will be clearly understood by those skilled in the art from the following description.

### [Description of Drawings]

FIG. 1 is a schematic diagram of a system for implementing an artificial intelligence-based method for predicting physical properties of a mixture according to one embodiment of the present disclosure.
FIG. 2 is a block diagram for describing a configuration of a device that performs the artificial intelligence-based method for predicting the physical properties of the mixture according to one embodiment of the present disclosure.
FIG. 3 is a block diagram for describing the artificial intelligence-based method for predicting the physical properties of the mixture according to one embodiment of the present disclosure.
FIG. 4 is a block diagram for describing a configuration and operation of an artificial intelligence (AI) model for predicting the physical properties of the mixture according to one embodiment of the present disclosure.
FIG. 5 is a diagram for describing a training/inference method of the AI model for predicting the physical properties of the mixture according to one embodiment of the present disclosure.
FIGS. 6A and 6B are diagrams for describing a training method of an AI model that extracts feature data of materials constituting the mixture according to one embodiment of the present disclosure.
FIGS. 7A, 7B, and 7C are diagrams for describing type-specific performance of the AI models according to one embodiment of the present disclosure.
FIGS. 8A, 8B, and 8C are diagrams for describing structures of the AI models for predicting the physical properties of the mixture according to one embodiment of the present disclosure.
FIGS. 9A, 9B, and 9C are diagrams for describing training/inference methods of the AI models for predicting the physical properties of the mixture according to one embodiment of the present disclosure.

### [Modes of the Invention]

The same reference numerals refer to the same components throughout the present disclosure. The present disclosure does not describe all elements of the embodiments, and common content in the art to which the present disclosure pertains or content that overlaps between the embodiments will be is omitted. Terms "unit," "module," "member," and "block" used in the specification may be implemented as software or hardware, and according to the embodiments, a plurality of "units," "modules," "members," and "blocks" may be implemented as one component, or one "unit," "module," "member," and "block" may also include a plurality of components.

Throughout the specification, when a first component is described as being "connected" to a second component, this includes not only a case in which the first component is directly connected to the second component but also a case in which the first component is indirectly connected to the second component, and the indirect connection includes connection through a wireless communication network.

In addition, when a certain portion is described as "including," a certain component, this means further including other components rather than precluding other components unless specifically stated otherwise.

Throughout the specification, when a first member is described as being positioned "on" a second member, this includes both a case in which the first member is in contact with the second member and a case in which a third member is present between the two members.

Terms such as first and second are used to distinguish one component from another, and the components are not limited by the above-described terms.

A singular expression includes plural expressions unless the context clearly dictates otherwise.

In each operation, identification symbols are used for convenience of description, and the identification symbols do not describe the sequence of each operation, and each operation may be performed in a different sequence from the specified sequence unless a specific sequence is clearly described in context.

Hereinafter, the operation principles and embodiments of the present disclosure will be described with reference to the accompanying drawings.

'A device for predicting physical properties of a mixture including two or more types of materials according to the present disclosure' in the present specification includes all of various devices that can perform computational processing and provide results to a user. For example, the device for predicting the physical properties of the mixture including two or more types of materials according to the present disclosure may include all of a computer, a server device, and a portable terminal, or may be in a form of any one of them.

Here, the computer may include, for example, a notebook, a desktop, a laptop, a tablet PC, a slate PC, etc., which are equipped with a web browser.

The server device is a server that processes information in communication with an external device, and may include an application server, a computing server, a database server, a file server, a game server, a mail server, a proxy server, and a web server.

The portable terminal is, for example, a wireless communication device ensuring portability and mobility and may include all kinds of handheld-based wireless communication devices such as a personal communication system (PCS), a global system for mobile communications (GSM), a personal digital cellular (PDC), a personal handyphone system (PHS), a personal digital assistant (PDA), international mobile telecommunication-2000 (IMT-2000), code division multiple access-2000 (CDMA-2000), w-code division multiple access (W-CDMA), a wireless broadband internet (WiBro) terminal, a smart phone, and wearable devices such as a watch, a ring, a bracelet, an anklet, a necklace, glasses, contact lenses, or a head-mounted device (HMD).

In addition, in describing the present disclosure, the mixture may include an electrolyte of a battery, but is not limited thereto, and may mean various types of mixtures including a plurality of materials.

Hereinafter, embodiments of the present invention will be described in detail with reference to the accompanying drawings.

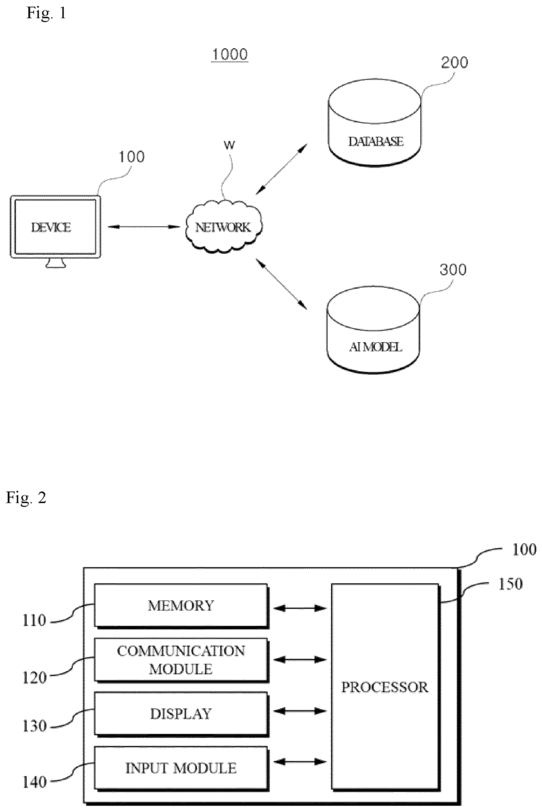

FIG. 1 is a schematic diagram of a system 1000 for implementing a method for predicting physical properties of a mixture including a plurality of materials according to one embodiment of the present disclosure.

As shown in FIG. 1, a system 1000 for implementing the method of predicting the physical properties of the mixture including the plurality of materials may include a device 100, a database 200, and an artificial intelligence (AI) model 300.

The device 100, the database 200, and the AI model 300 included in the system 1000 may perform communication via a network W. Here, the network W may include a wired network and a wireless network. For example, the network may include various networks such as a local area network (LAN), a metropolitan area network (MAN), and a wide area network (WAN).

In addition, the network W may include the well-known World Wide Web (WWW). However, the network W according to the present disclosure is not limited to the above-listed networks and may include a well-known wireless data network, a well-known telephone network, or a well-known wired and wireless television network as at least a portion thereof.

The device 100 may predict the physical properties of the mixture including the plurality of materials based on the AI model 300. That is, the device 100 may predict the physical properties of the mixture based on the AI model that may consider interaction between materials constituting the mixture.

Specifically, the device may and obtain first feature data associated with each of the corresponding materials by inputting material information of each of the plurality of materials constituting the mixture into a first AI model.

Here, the first feature data may include molecular feature data (e.g., a molecular feature vector, etc.) of each of the corresponding materials. The molecular feature data may be based on information obtained from an external device but is not limited thereto and may also be obtained through calculation (e.g., Python program, RdKit, Mordred, etc.).

In addition, the material information may include chemical information, and the chemical information may include various types of molecular information (e.g., a simplified molecular-input line-entry system (SMILES), an international chemical identifier (INCHI), a self-referencing embedded string (SELFIES)), and the like. As an example, the device may obtain the first feature data associated with each of the corresponding materials by inputting molecular information of each of the plurality of materials constituting the mixture into the first AI model.

As another example, the chemical information may include at least one of atom properties or bond properties related to the mixture.

In addition, the device may obtain physical property prediction information associated with the mixture by inputting the first feature data into a second AI model. As another example, the device may obtain the physical property prediction information associated with the mixture by inputting the first feature data and component ratio information of the plurality of materials in the mixture into the second AI model. Here, the physical property prediction information may include Gibbs free energy prediction information but is not limited thereto.

The device may predict the physical properties of the mixture based on the obtained physical property prediction information (e.g., the Gibbs free energy prediction information).

As an example, a difference (e.g., a root mean square error (RMSE)) between the Gibbs free energy prediction information obtained by the device and actual Gibbs free energy may be less than 0.02. However, the present disclosure is not limited thereto, and the RMSE value may vary depending on an experimental environment/the number of training times of the model, etc.

The database 200 may store various training data for training the AI model 300. In addition, the database 200 may store the chemical information of two or more materials constituting the mixture. In addition, the database 200 may store the component ratio information of the plurality of materials in the corresponding mixture.

FIG. 1 shows a case in which the database 200 is implemented outside the device 100. In this case, the database 200 may be connected to the device 100 in a wired or wireless communication manner. However, this is only one embodiment, and the database 200 may also be implemented as one component of the device 100.

The AI model 300 may include a model trained for predicting the physical properties of the mixture including the plurality of materials. Specifically, the AI model 300 may include the first AI model trained to extract the feature data of each material constituting the mixture and the second AI model trained to extract the physical property prediction information associated with the mixture.

As an example, the first AI model and the second AI model may be trained in an end-to-end manner. The end-to-end training manner is a method of training an AI model to process at once from an input to an output without a pipeline network. Here, the pipeline network is a partial network forming the entire network. That is, when trained in an end-to-end manner, the first AI model and the second AI model may be implemented as a single AI model.

Accordingly, the device may obtain the Gibbs free energy prediction information associated with the mixture by inputting the feature data of each material constituting the mixture into the AI model trained in an end-to-end manner.

However, this is only one embodiment, and the first AI model and the second AI model may be implemented as separate models (or separate pipeline networks) and each model may be trained separately.

FIG. 1 shows a case in which the AI model 300 is implemented outside the device 100 (e.g., implemented in a cloud-based manner), but the AI model 300 is not limited thereto, and may be implemented as one component of the device 100.

FIG. 2 is a block diagram for describing a configuration of a device that performs an artificial intelligence-based method for predicting the physical properties of the mixture according to one embodiment of the present disclosure.

As shown in FIG. 2, the device 100 may include a memory 110, a communication module 120, a display 130, an input module 140, and a processor 150. However, the present disclosure is not limited thereto, and software and hardware components of the device 100 may be modified/added/omitted according to a required operation within a scope obvious to those skilled in the art.

The memory 110 may store data supporting various functions of the device 100 and a program for operating the processor 150, store input/output data (e.g., music files, still images, videos, etc.), and store a plurality of application programs or applications that are driven on the present device, and data and commands for operating the device 100. At least some of the application programs may be downloaded from an external server via wireless communication.

Such memory 110 may include at least one type of storage medium among a flash memory type, a hard disk type, a solid state disk type (SSD type), a silicon disk drive type (SDD type), a multimedia card micro type, a card-type memory (e.g., an SD or XD memory), a random access memory (RAM), a static random access memory (SRAM), a read-only memory (ROM), an electrically erasable programmable ROM (EEPROM), a programmable ROM (PROM), a magnetic memory, a magnetic disk, and an optical disk.

In addition, the memory 110 may include a database that is separate from the present device but is connected in a wired or wireless communication manner. That is, the database 200 shown in FIG. 1 may be implemented as one component of the memory 110.

The communication module 120 may include one or more components that enable communication with an external device, and may include at least one of, for example, a broadcasting reception module, a wired communication module, a wireless communication module, a short-range communication module, or a location information module.

The wired communication module may include not only various wired communication modules such as a LAN module, a WAN module, and a value added network (VAN) module, but also various cable communication modules such as a universal serial bus (USB), a high definition multimedia interface (HDMI), a digital visual interface (DVI), a recommended standard 232 (RS-232), power line communication, and a plain old telephone service (POTS).

In addition to the WiFi module and the wireless broadband (WiBro) module, the wireless communication module may include a wireless communication module for supporting various wireless communication methods such as global system for mobile communication (GSM), code division multiple access (CDMA), wideband CDMA (WCDMA), universal mobile telecommunications system (UMTS), time division multiple access (TDMA), long term evolution (LTE), 4G, 5G, or 6G.

The display 130 displays (outputs) information (e.g., the feature data output from each AI model/ the physical property prediction information (e.g., the Gibbs free energy prediction information)/physical property prediction result information of the mixture, etc.) processed by the device 100.

For example, the display may display execution screen information of an application program (e.g., an application) driven on the device 100, or user interface (UI) or graphic user interface (GUI) information according to such execution screen information. The type of UI output by the display 130 will be described below.

The input module 140 is for receiving information from a user, and when receiving information through a user input unit, the processor 150 may control the operation of the device 100 to correspond to the input information.

The input module 140 may include a hardware physical key (e.g., a button located on at least one of a front surface, a back surface, and a side surface of the present device, a dome switch, a jog wheel, a jog switch, etc.) and a software touch key. As an example, the touch key may be formed as a virtual key, a soft key, or a visual key that is displayed on a touchscreen type of the display 130 through software processing or may be formed as the touch key disposed a portion other than the touchscreen. Meanwhile, the virtual key or visual key may have various forms and may be displayed on the touchscreen, and may be formed as, for example, a graphic, text, an icon, a video, or a combination thereof.

The processor 150 may be implemented with a memory that stores data for an algorithm for controlling the operations of the components in the device 100 or a program that reproduces the algorithm, and at least one processor (not shown) that performs the above-described operation using the data stored in the memory. In this case, each of the memory and the processor may be implemented as a separate chip. Alternatively, the memory and the processor may also be implemented as a single chip.

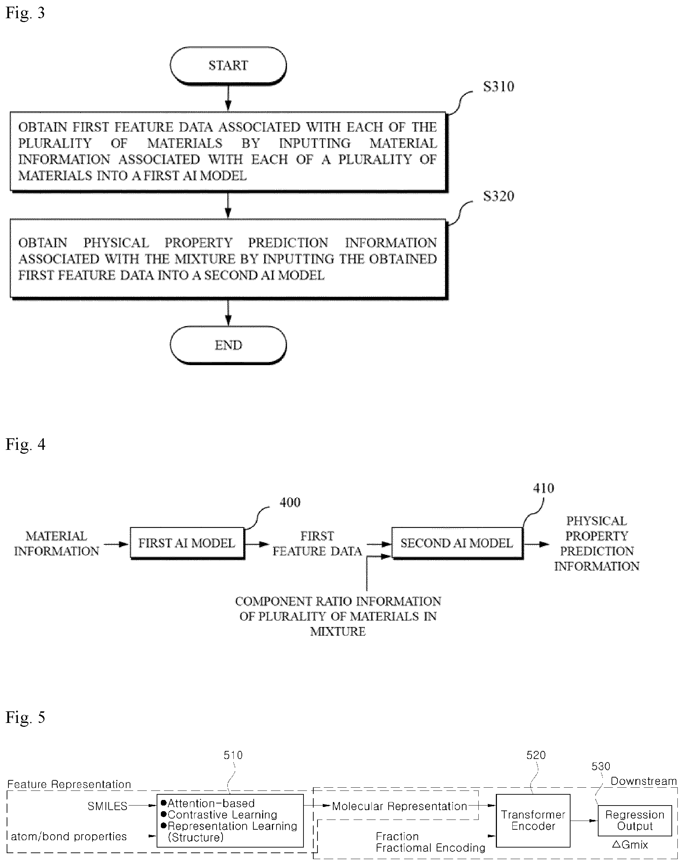

In addition, the processor 150 may control the operations of the components in the device 100 by combining any one or a plurality of the above-described components in order to implement various embodiments according to the present disclosure, which will be described in FIGS. 3 to 8 below, on the device 100.

FIG. 3 is a block diagram illustrating the artificial intelligence-based method for predicting the physical properties of the mixture according to one embodiment of the present disclosure.

The device may obtain first feature data associated with each of the plurality of materials (S310) by inputting material information of each of a plurality of materials into a first AI model.

Here, the material information may include chemical information. In addition, the chemical information may include molecular information (e.g., a SMILES, an INCHI, or a SELFIES) of each of the plurality of materials.

The first AI model may include a model trained to output the first feature data (e.g., molecular feature data of each of the materials, etc.) of each of the corresponding materials. As shown in FIG. 4, input data of the first AI model 400 may include the material information of each of the plurality of materials constituting the mixture, and output data of the first AI model 400 may include the first feature data.

For example, as shown in FIG. 5, the first AI model 500 may include at least one of an attention-based model, a molecular contrastive learning-based model, or a representative learning-based model.

Here, the attention-based model may be trained to extract feature data of chemical information for a specific material based on physicochemical properties (e.g., at least one of polarizability information and hydrophobicity information (e.g., sLogP)) of the specific material. That is, the attention-based model may be trained to extract the first feature data from the final layer after training based on the specific physical property.

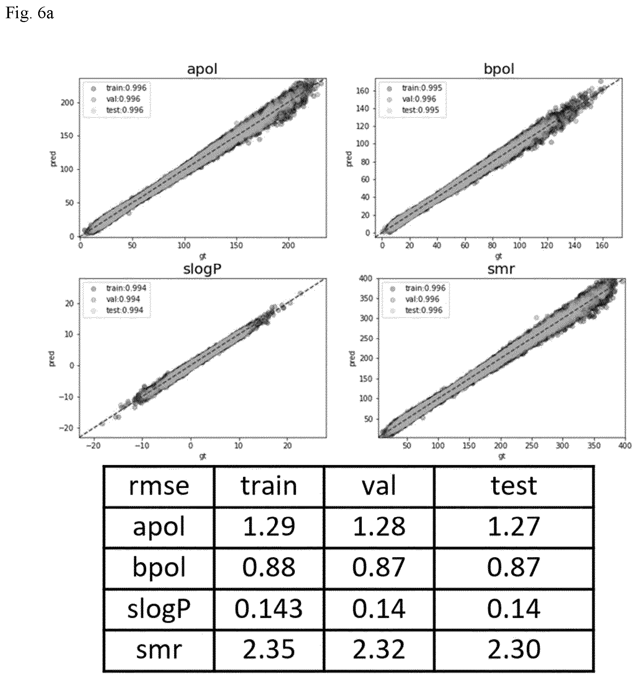

FIG. 6A shows an example of the result data of training of the attention-based model. The attention-based model may be trained to extract the feature data of the chemical information for the specific material based on information that is highly relevant to the Gibbs free energy (e.g., a Wildman-Crippen LogP property (sLogP), a Wildman-Crippen MR property (smr), a bond polarizability property (bpol), or an atomic polarizability property (apol)), etc.

The molecular contrastive learning-based model may be trained to align molecules with similar structures on a latent space. That is, the molecular contrastive learning-based model may be trained to calculate how many molecules with similar structures exist nearby and to align molecules with similar structures based on the result of the calculation. The latent space is a space of feature data (or molecular feature vector) of chemical information of a specific material.

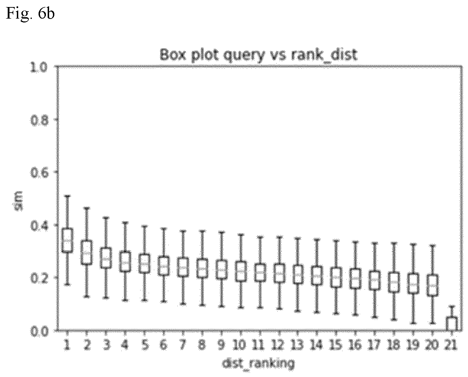

For example, FIG. 6B shows an example of the result data of training of the molecular contrastive learning-based model. The x-axis of the graph shown in FIG. 6B indicates similarity between molecules, and the y-axis indicates a distance. According to the graph shown in FIG. 6B, as the distance between molecules increases, the similarity between molecules tends to decrease.

As another example, the first AI model may be trained to update second feature data extracted from two-dimensional graph information of a specific material based on third feature data extracted from three-dimensional structural characteristics (e.g., a plurality of spatial arrangement conformers for the specific material) of the specific material. In addition, the first AI model may be trained to output the first feature data based on the updated data. In this case, the first AI model may be trained to consider the plurality of spatial arrangement conformers for the specific material.

Specifically, when the chemical information implemented as the two-dimensional graph information for the specific material is input, the pre-trained first AI model may process the second feature data extracted from the corresponding chemical information to be updated by reflecting the third feature data extracted from the three-dimensional structural characteristics for the specific material. Accordingly, the second feature data may be updated to include the three-dimensional structural characteristics. In addition, the pre-trained first AI model may output the first feature data based on the updated data.

As another example of the present disclosure, the chemical information that is one example of the material information may include at least one of atom properties or bond properties related to the mixture. For example, the device may obtain the first feature data by inputting, into the first AI model, the molecular information (e.g., a SMILES, an INCHI, or a SELFIES) of each of the plurality of materials. That is, the first AI model may be trained to output the first feature data based on the molecular information related to the mixture (considering both atom properties and bond properties).

Only the molecular information may be input into the first AI model, but the present disclosure is not limited thereto, and at least one of the atom properties or the bond properties may be selectively input.

The device may obtain physical property prediction information associated with the mixture (S320) by inputting the obtained first feature data into a second AI model.

Specifically, the device may obtain the physical property prediction information associated with the mixture by inputting the first feature data and component ratio information of the plurality of materials in the mixture into the second AI model. Here, the component ratio information (or fraction information) of the plurality of materials in the mixture may be information encoded in a predefined manner.

That is, as shown in FIG. 4, input data of the second AI model 410 may include at least one of the first feature data or the component ratio information of the plurality of materials. In addition, output data of the second AI model 410 may include the component ratio information of the plurality of materials in the mixture.

In addition, the second AI model may be trained to output the physical property prediction information for the mixture (e.g., Gibbs free energy prediction information) based on the component ratio information of the plurality of materials in the mixture and the first feature data.

As an example, the second AI model may include at least one of a multi-layer perceptron (MLP)-based model, a ResNet model, or a transformer encoder model. The device may obtain prediction information associated with the physical properties of the mixture based on the physical property prediction information (e.g., the Gibbs free energy prediction information).

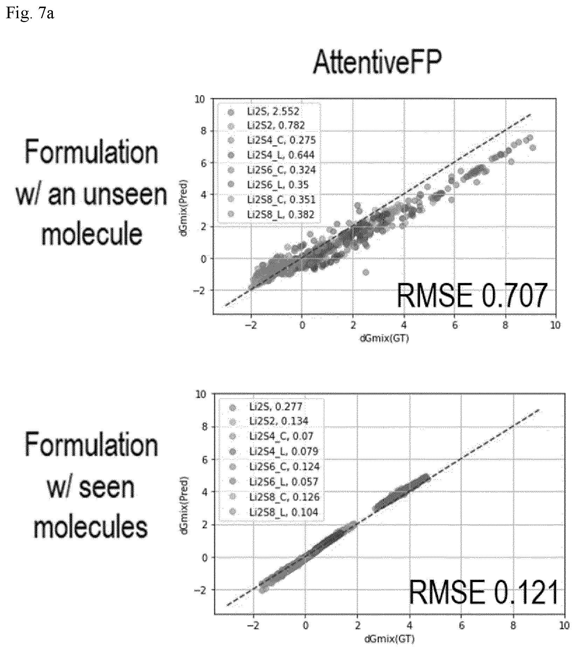

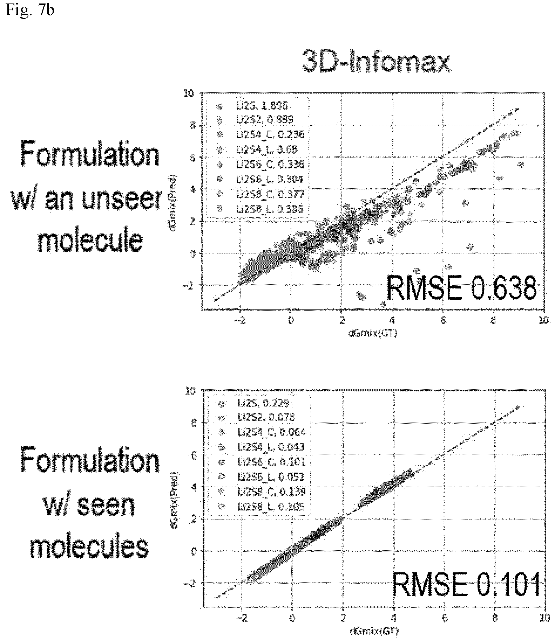

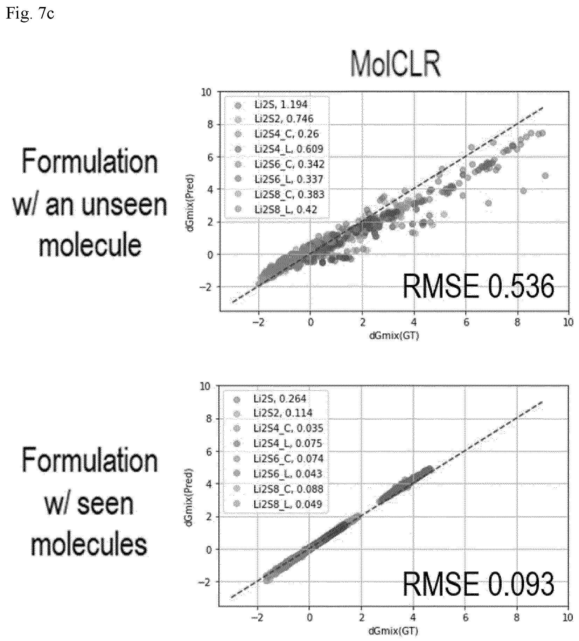

FIGS. 7A, 7B, and 7C are diagrams for describing type-specific performance of the AI models according to one embodiment of the present disclosure.

FIG. 7A shows an example of the result of extracting the feature data according to the first AI model when the first AI model includes the attention-based model.

FIG. 7B shows an example of the result of extracting the feature data according to the first AI model when the first AI model is trained to update the second feature data extracted from the two-dimensional graph information of the specific material based on the third feature data extracted from the three-dimensional structural characteristics of the specific material.

FIG. 7C shows an example of the result of extracting the feature data according to the first AI model when the first AI model includes the molecular contrastive learning-based model.

The upper image of each of FIGS. 7A, 7B, and 7C shows the result of extracting the feature data for the mixture, which was not a training target during the training process, by the first AI model. The lower image of each of FIGS. 7A, 7B, and 7C shows the result of extracting the feature data for the mixture, which was the training target during the training process, by the first AI model.

When comparing the upper and lower images of each of FIGS. 7A, 7B, and 7C, it can be confirmed that an RMSE value decreases in a case in which the first AI model extracts the feature data for the mixture that was the training target during the training process compared to the other case.

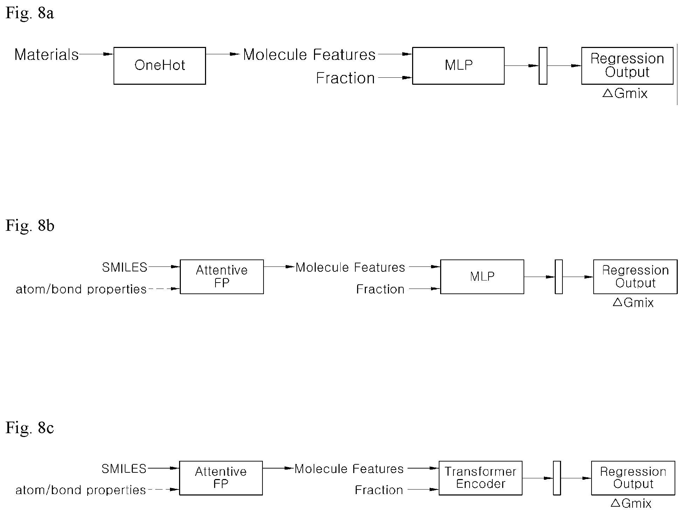

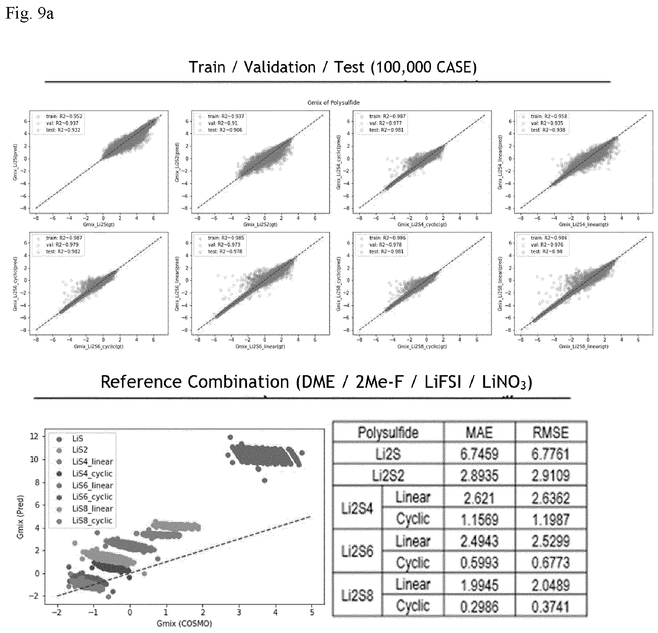

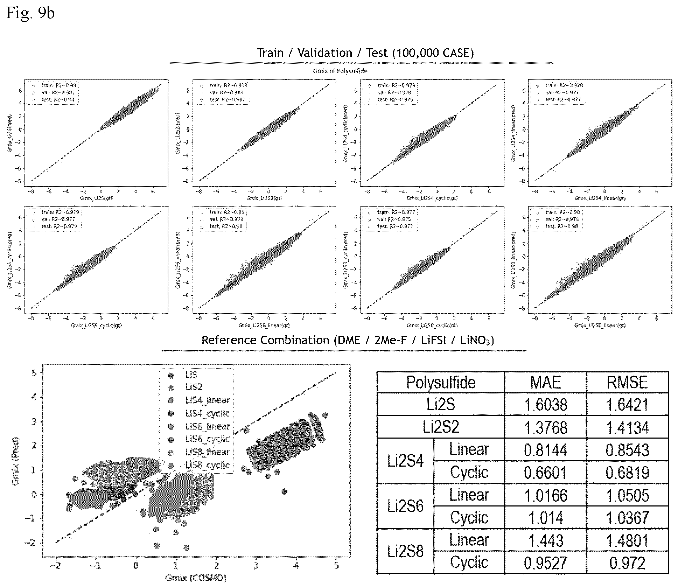

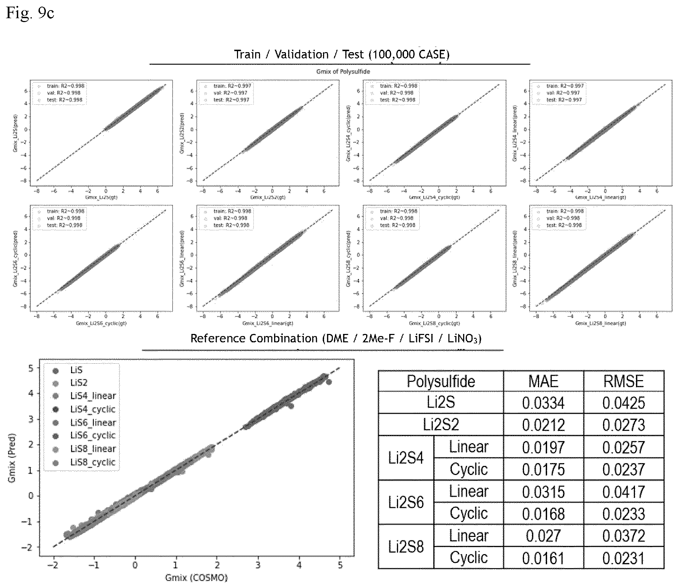

FIGS. 8A, 8B, and 8C are diagrams illustrating structures of the AI models for predicting the physical properties of the mixture according to one embodiment of the present disclosure. In addition, FIGS. 9A, 9B, and 9C are diagrams illustrating training/inference methods of the AI models for predicting the physical properties of the mixture according to an embodiment of the present disclosure.

In FIGS. 8B and 8C, cases in which the first AI model is implemented as an attentive FP model (i.e., an example of an attention-based model) are shown, but the present disclosure is not limited thereto. The first AI model may be implemented as an AI model that may extract feature data of a material, such as the molecular contrastive learning-based model.

In addition, in FIGS. 8B and 8C, the operations of inputting, into the first AI model, the molecular information (e.g., a SMILES, an INCHI, or a SELFIES) as the material information of the material constituting the mixture are shown, but the present disclosure is not limited thereto. The input data for the first AI model may be composed of only the material information.

As another example, at least one of the atom properties or the bond properties for the mixture may be selectively input into the first AI model. As still another example, at least one of the atom properties or the bond properties for the mixture may be obtained based on the molecular information.

In addition, in FIGS. 8A, 8B, and 8C, the operations in which the second AI model (e.g., the transformer encoder) outputs the Gibbs free energy prediction information are shown, but the present disclosure is not limited thereto and the second AI model may output various types of the physical property prediction information.

FIG. 8A shows a process of extracting the physical property prediction information associated with the mixture through a one hot encoding model and the MLP-based model. In FIG. 8B, when the material information of each of the plurality of materials constituting the mixture is input, the one hot encoding model may be trained to output molecular feature data mapped to the input material information. In this case, the one hot encoding model may be trained to output information pre-mapped to the chemical information, rather than extracting the feature data from the material information (e.g., the chemical information) of the materials constituting the mixture.

FIG. 8C shows a process of extracting the physical property prediction information (e.g., the Gibbs free energy prediction information) associated with the mixture through the first AI model including the attention-based model and the second AI model based on the MLP-based model. FIG. 8C also shows a process of extracting the physical property prediction information (e.g., the Gibbs free energy prediction information) associated with the mixture through the first AI model including the attention-based model and the transformer encoder model.

As described above, unlike FIGS. 8B and 8C, FIG. 8A does not include a process of extracting the first feature data associated with each of the plurality of materials constituting the mixture through the first AI model.

FIGS. 9A, 9B, and 9C show result data according to FIGS. 8A, 8B, and 8C.

When comparing FIGS. 9A with 9B, the performance difference that occurs due to utilizing the feature data extracted from the molecular information (e.g., a SMILES, an INCHI, or a SELFIES) of the materials constituting the mixture can be confirmed. When utilizing the feature data extracted from the material information of the materials, difference values between actual physical property information (e.g., Gibbs free energy) and the physical property prediction information (e.g., predicted Gibbs free energy information) obtained through the AI model may decrease.

When comparing FIGS. 9A with 9B, the performance difference between the MLP-based model and the transformer encoder model can be confirmed. When utilizing the transformer encoder model, more accurate prediction information can be output than when utilizing the MLP-based model.

Meanwhile, disclosed embodiments may be implemented in the form of a recording medium in which computer-executable commands are stored. The commands may be stored in the form of program code, and when executed by a processor, program modules are generated to perform operations of the disclosed embodiments. The recording medium may be implemented as a computer-readable recording medium.

The computer-readable recording medium includes all types of recording media in which computer-decodable commands are stored. For example, there may be a ROM, a RAM, a magnetic tape, a magnetic disk, a flash memory, an optical data storage device, and the like.

As described above, the disclosed embodiments have been described with reference to the accompanying drawings. Those skilled in the art to which the present disclosure pertains will understand that the present disclosure may be implemented in different forms from the disclosed embodiments without departing from the technical spirit or essential features of the present disclosure. The disclosed embodiments are illustrative and should not be construed as being limited.

## Claims

1. A device for predicting physical properties of a mixture including a plurality of materials, the device comprising:
a memory in which a first artificial intelligence (AI) model trained to output first feature data associated with material information, and a second AI model trained to output physical property prediction information associated with the first feature data are stored; and
a processor configured to execute the first AI model and the second AI model,
wherein the processor is configured to:
obtain first feature data associated with each of the plurality of materials by inputting, into the first AI model, material information of each of the plurality of materials; and
obtain physical property prediction information associated with the mixture by inputting the first feature data into the second AI model.

2. The device of claim 1, wherein the first AI model includes an attention-based model, and
the attention-based model is trained to extract feature data of a specific material based on at least one of polarizability information and hydrophobicity information of the specific material.

3. The device of claim 1, wherein the first AI model includes a molecular contrastive learning-based model, and
the molecular contrastive learning-based model is trained to align molecules with similar structures on a latent space.

4. The device of claim 1, wherein the first AI model is trained to update second feature data extracted from two-dimensional graph information of a specific material based on third feature data extracted from three-dimensional structural characteristics of the specific material, and is trained to output the first feature data based on the updated data.

5. The device of claim 4, wherein the three-dimensional structural characteristics of the specific material include a plurality of spatial arrangement conformers of the specific material.

6. The device of claim 1, wherein the second AI model includes a multi-layer perceptron-based model.

7. The device of claim 1, wherein the second AI model includes a transformer encoder model.

8. The device of claim 1, wherein the processor is configured to:
obtain physical property prediction information associated with the mixture by inputting the first feature data and component ratio information of the plurality of materials in the mixture into the second AI model.

9. The device of claim 1, wherein the first AI model and the second AI model are trained in an end-to-end training manner.

10. The device of claim 1, wherein the material information includes chemical information,
the chemical information includes molecular information of each of the plurality of materials, and
the molecular information includes at least one of a simplified molecular-input line-entry system (SMILES), an international chemical identifier (INCHI), or a self-referencing embedded string (SELFIES).

11. The device of claim 10, wherein the chemical information includes at least one of atom properties or bond properties related to the mixture.

12. The device of claim 1, wherein the first feature data includes molecular feature data of each of the plurality of materials.

13. The device of claim 1, wherein the physical property prediction information includes Gibbs free energy prediction information.

14. A method for predicting physical properties of a mixture including a plurality of materials, performed by a device, the method comprising:
obtaining first feature data associated with each of the plurality of materials by inputting material information associated with each of the plurality of materials into a first AI model that has trained first feature data associated with material information; and
obtaining physical property prediction information associated with the mixture by inputting the first feature data into a second AI model that has trained physical property prediction information associated with the first feature data.

15. A computer program that is stored in a computer-readable recording medium coupled with a hardware device to execute the method for predicting the physical properties of the mixture including the plurality of materials of claim 14.
